(19) Europäisches Patentamt
European Patent Office
Office européen des brevets

(11) Veröffentlichungsnummer: **0 325 954**
**A2**

(12) # EUROPÄISCHE PATENTANMELDUNG

(21) Anmeldenummer: **89100456.6**

(22) Anmeldetag: **12.01.89**

(51) Int. Cl.⁴: **C12P 17/02 , C12P 41/00 , //(C12P17/02,C12R1:38)**

(30) Priorität: **26.01.88 US 148469**

(43) Veröffentlichungstag der Anmeldung: **02.08.89 Patentblatt 89/31**

(84) Benannte Vertragsstaaten: **AT BE CH DE FR GB IT LI NL**

(71) Anmelder: **F. HOFFMANN-LA ROCHE & CO. Aktiengesellschaft**
**Postfach 3255**
**CH-4002 Basel(CH)**

(72) Erfinder: **Coffen, David Llewellyn**
**270 Ridgewood Avenue**
**Glenridge, N.J. 07028(US)**
Erfinder: **Kalaritis, Panayiotis**
**25 Evergreen Avenue**
**New Providence, N.J. 07974(US)**
Erfinder: **Partridge, John Joseph**
**620 Airport Road**
**Chapel Hill, No. Carolina 27514(US)**

(74) Vertreter: **Kellenberger, Marcus, Dr. et al**
**Grenzacherstrasse 124 Postfach 3255**
**CH-4002 Basel(CH)**

(54) **Verfahren zur Herstellung optisch reiner Hydroxy-substituierter Benzopyrancarbonsäuren und -ester.**

(57) Enantiomer reine (2R)-Hydroxy-substituierte Benzopyran-2-carbonsäureester und (2S)-Hydroxy-substituierte Benzopyran-2-carbonsäuren der Formeln

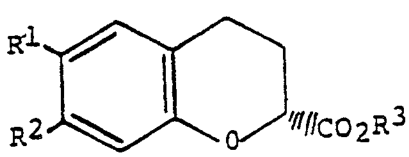

IA

und

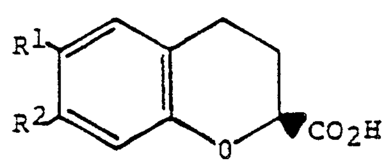

IB

worin einer von $R^1$ und $R^2$ Hydroxy und der andere Wasserstoff und $R^3$ Alkyl, Aryl oder Aralkyl bedeuten, werden durch eine Pseudomonas Lipase-katalysierte selektive Hydrolyse in Lösung oder in Suspension in einem wässrigen oder wässrig-organischen Medium bei einem kontrollierten pH-Wert zwischen ungefähr 5 und ungefähr 10 aus racemischen (2RS)-Hydroxy-substituierten Benzopyran-2-carbonsäureestern der Formel

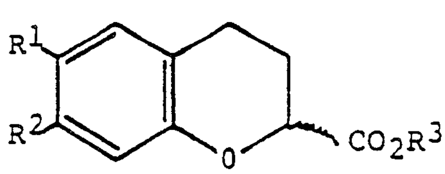

II

worin $R^1$, $R^2$ und $R^3$ die oben angegebene Bedeutung besitzen,

EP 0 325 954 A2

hergestellt.

## Verfahren zur Herstellung optisch reiner Hydroxy-substituierter Benzopyrancarbonsäuren und -ester

Die vorliegende Erfindung betrifft ein neues Verfahren zur Herstellung optisch reiner Hydroxy-substituierter Benzopyran-2-carbonsäuren und -ester der allgemeinen Formeln

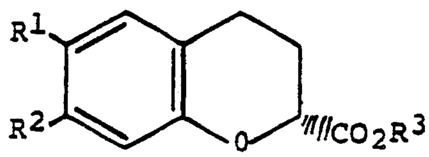

IA
(R)

und

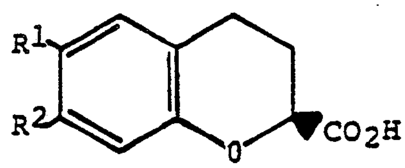

IB
(S)

worin einer von $R^1$ und $R^2$ Hydroxy und der andere Wasserstoff und $R^3$ Alkyl, Aryl oder Aralkyl bedeuten, durch enzymatisch kinetische Aufspaltung von racemischen (2RS)-3,4-Dihydro-6- oder 7-hydroxy-2H-1-benzopyran-2-carbonsäureestern der allgemeinen Formel

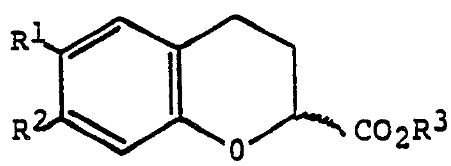

II
(R,S)

worin $R^1$, $R^2$ und $R^3$ die oben angegebene Bedeutung besitzen.

Der in dieser Beschreibung verwendete Ausdruck "Alkyl" betrifft geradkettige und verzweigte Alkylgruppen, vorzugsweise Niederalkylgruppen mit 1-8 Kohlenstoffatomen, wie Methyl, Aethyl, Propyl, Isopropyl, Butyl und dergleichen.

Der in dieser Beschreibung ebenfalls verwendete Ausdruck "Aryl" betrifft monocyclische aromatische Kohlenwasserstoffgruppen, wie Phenyl, und polycyclische Arylgruppen, wie Naphthyl, Anthryl, Phenanthryl und dergleichen. Die bevorzugten Arylgruppen sind monocyclische Arylgruppen, insbesondere Phenyl.

Der Ausdruck "Aralkyl" betrifft geradkettige und verzweigte Alkylgruppen, vorzugsweise solche mit 1-8 Kohlenstoffatomen, welche in einer oben definierten Arylgruppe enden.

Jede der oben erwähnten Alkyl-, Aryl- oder Aralkylgruppen kann gegebenenfalls in einer oder mehreren Stellungen durch eine Reihe von Substituenten, wie Halogen, Alkoxy, Aryloxy, Thioalkoxy, Thioaryloxy und Alkyl, vorzugsweise Halogen (Chlor, Brom, Fluor oder Jod), substituiert sein.

In den in der vorliegenden Beschreibung angegebenen Formeln bedeutet eine dicke, spitzzulaufende Linie (▲) einen Substituenten mit $\beta$-Orientierung (d.h. oberhalb der Ebene des Moleküls bzw. der Seite), eine gestrichelte Linie ( ▲ ) einen Substituenten mit $\alpha$-Orientierung (d.h. unterhalb der Ebene des Moleküls bzw. der Seite) und eine Wellenlinie ( ≀ ) einen Substituenten mit $\alpha$- oder $\beta$-Orientierung oder Gemische dieser Isomeren.

Erfindungsgemäss wurde gefunden, dass das 2S-Enantiomere der Formel II selektiv zum 2S-Enantiomeren der Formel IB hydrolysiert wird, wenn man das racemische Gemisch der Formel II einer enzymatischen Hydrolyse unter Verwendung eines bakteriellen Lipase-Enzyms, welches aus einer Pseudomonas Spezies erhalten wurde, unterwirft. Die enzymtisch-kinetische Aufspaltung kann auch verwendet werden, um ein racemisches Gemisch der Formel II in das 2R-Enantiomere der Formel IA überzuführen.

Die erfindungsgemässe enzymtische Hydrolyse führt demnach zum 2R-Enantiomeren der Formel IA im Gemisch mit dem 2S-Enantiomeren der Formel IB. Diese Verbindungen können danach leicht nach

üblichen Methoden getrennt werden.

Die Spezifität gewisser Mikroorganismen oder gewisser Enzyme, welche aus Mikroorganismen gewonnen wurden, ermöglicht deren potentielle Verwendung zur Herstellung von enantiomer reinen Zwischenprodukten aus racemischen Gemischen. Das erwünschte enantiomere Molekül kann danach in die Zielverbindung übergeführt werden. Die durch einen Mikroorganismus oder ein Enzym katalysierte Aufspaltung von Isomeren ergibt eine attraktive Alternative zu den mehr traditionellen und kostspieligen Methoden, wie chemische Aufspaltung und Hochdruck-Flüssigchromatographie diastereomerer Derivate.

Kato et al. haben berichtet, dass ein bekanntes Bakterium, nämliche Corynebacterium equi IFO 3730, die Eigenschaft besitzt, verschiedene Ester enantioselektiv zu hydrolysieren (Tetrahedron Letters., Band 28, Nr. 12, 1987, Seiten 1303-1306). In ihrer Studie wurde der Mikroorganismus für die asymmetrische Hydrolyse 2-Benzyloxy-substituierter Alkan- und Arylalkancarbonsäureester eingesetzt, wobei eine Suspension gewachsener Zellen von Corynebacterium equi und ein verlängerter (z.B. 24 Stunden) Fermentationsprozess zur Anwendung kamen. Die nicht reagierten Niederalkylester wurden in der optisch aktiven S-Form wiedergewonnen, und zwar in einer hohen enantiomeren Reinheit (über 99% e.R.). Es wurde auch festgestellt, dass der Austausch des Alkyl-oder Alkenylrests des Substrates mit einer Phenylmethylgruppe eine Umkehrung der Stereoselektivität verursachte; d.h. die Verbindung fiel in der optisch aktiven R-Form an, und zwar ebenfalls in hoher enantiomerer Reinheit.

Kitazume et al. haben ein Verfahren zur asymmetrischen Hydrolyse von 2-Fluor-2-methylmalonsäurediestern mit Esterase aus Schweineleber beschrieben, gemäss welchem die optisch aktiven (-)-2-Fluor-2-methylmalonsäuremonoester erhalten wurden, jedoch in niedriger enantiomerer Reinheit. Beschrieben wurde auch eine mikrobielle Hydrolyse von 2-Fluor-2-substituierten Malonsäurediestern mit Esterase und Cellulase, wobei die optisch aktiven (+)- oder (-)-2-Fluor-2-substituierten Malonsäuremonoester erhalten wurden (J. Org. Chem., 51, 1986, Seiten 1003-1006).

Gu et al. haben berichtet, dass optisch aktive 3-Benzoylthio-2-methylpropionsäuren durch mikrobielle Lipase-katalysierte enantioselektive Hydrolyse ihrer entsprechenden Ester hergestellt werden können. Enantioselektivität bezüglich des stereochemisch bevorzugten S-Isomeren war jedoch mit allen eingesetzten Lipasen schlecht, weshalb zum Erreichen einer höheren Stereoselektivität eine Aenderung in der Struktur des Aroylthio-Teils des Substrats notwendig war. Insbesondere konnten durch Einführung von Methoxygruppen in den Phenylring in den Stellungen 3 und 5 die Stereospezifität von Lipase, welche aus Mucor meihei erhalten wurde, verbessert werden (Tetrahedron Letters, Band 27, Nr. 43, 1986, Seiten 5203-5206).

Iuchijima et al. haben ein Verfahren zur Herstellung optisch aktiver 2-Chlor- und 2-Brom-substituierter Alkylester und Säuren durch asymmetrische Hydrolyse racemischer Gemische der Ester beschrieben, wobei die Mikroorganismen Rizopus, Mucor, Aspergillus, Candida, Pseudomonas, Alcaligenes, Achromobacter und Bacillus, oder aus diesen Mikroorganismen gewonnene Enzyme verwendet wurden (publizierte Japanische Patentanmeldung [Kokai] Nr. 57-94.295 [1982]).

Ebenfalls in der Literatur beschrieben wurde die Candida Lipase-katalysierte, enantioselektive Hydrolyse von racemischem 2-Chlorpropionsäureoctylester zur R-Form der 2-Chlorpropionsäure (Cambou und Klibanov, Appl. Biochem. Biotech., 9, 1984, Seite 255).

U.S. Patentschrift Nr. 4,668,628 (Dahod et al.) beschreibt ein Verfahren zur enzymatischen Spaltung racemischer Gemische von partiell wasserlöslichen Estern, welches darin besteht, dass man das racemische Gemisch mit einem Candida Lipase-Enzym in Kontakt bringt, um dieses enzymatisch zu hydrolysieren. Ein spezifisches Beispiel ist die Candia Lipase-katalysierte Hydrolyse von D,L-2-Chlorpropionsäuremethylester.

Ein Nachteil Lipase-katalysierter kinetischer Spaltungen ist insbesondere die Tatsache, dass die Spezifität des Enzyms für ein gegebenes Substrat oft nicht vorausgesagt werden kann, da keine nützlichen Modelle existieren, welche für eine Lipase-katalysierte kinetische Aufspaltung eines potentiellen Substrates eine Voraussage bezüglich der Stereochemie ermöglichen.

Bei der Durchführung der erfindungsgemässen enzymatischen Aufspaltung wird die Verbindung der Formel II in Wasser oder einem Gemisch aus Wasser und einem organischen Lösungsmittel gelöst oder, falls notwendig, suspendiert.

Wenn die Verbindung der Formel II in Wasser suspendiert wird, können auch noch Emulgiermittel verwendet werden, um die Emulsionsbildung zu verbessern oder zu erleichtern, wobei konventionelle Emulgiermittel für diesen Zweck verwendet werden können.

Das organische Lösungsmittel bzw. die organischen Lösungsmittel, welche im gemischten System von Wasser und organischem Lösungsmittel verwendet werden, können mit Wasser vollständig mischbar sein, beispielsweise Methanol und Aceton, oder nur partiell, beispielsweise Acetonitril, Tetrahydrofuran, Aether und Toluol. Gewöhnlich wird volumenmässig mehr Wasser als organisches Lösungsmittel verwendet. Das

Volumenverhältnis von Wasser zu organischem Lösungsmittel liegt meistens im Bereich von etwa 1:1 bis etwa 10:1, vorzugsweise von etwa 3:1 bis etwa 9:1.

Die enzymatische Hydrolyse wird bei einem pH von ungefähr 5 bis ungefähr 10, vorzugsweise bei einem pH von ungefähr 7 bis ungefähr 9, durchgeführt. Um den pH-Wert des Reaktionsgemisches im vorgenannten Bereich halten zu können, kann jede übliche Methode verwendet werden. Unter den bevorzugten Methoden kann die Verwendung von Puffern oder die automatische Titration genannt werden.

Bei der Durchführung dieser enzymatischen Hydrolyse wird das racemische Gemisch der Formel II in einem wässrigen Medium gelöst oder anderweitig dispergiert und mit bakteriellem Lipase-Enzym umgesetzt. Im allgemeinen ist es bevorzugt, das Enzym in einer katalytisch wirksamen Menge zu verwenden. Unbestrittenermassen wird die zum Erhalten bester Resultate notwendige Bestimmung einer katalytisch wirksamen Menge eines bestimmten Enzyms von Faktoren abhängen, welche einem Fachmann geläufig sind. Diese Faktoren umfassen die Menge Ausgangsmaterial, die Herkunft des Enzyms, die Aktivität des Enzyms, die Reinheit des Enzyms und dergleichen. Man kann zwar einen Ueberschuss einer katalytisch wirksamen Menge des bakteriellen Lipase-Enzyms verwenden, doch wird durch die Verwendung eines grossen Ueberschusses an Enzym keine Verbesserung des Resultats erreicht.

Wie oben erwähnt, liefert die enzymatische Hydrolyse der racemischen Verbindung der Formel II die Verbindung der Formel IA im Gemisch mit der Verbindung der Formel IB. Diese Verbindungen können leicht getrennt werden, wenn die enzymatische Hydrolyse einmal gestoppt ist, und zwar durch sofortige Extraktion des Reaktionsmediums mit einem geeigneten organischen Lösungsmittel. Jede übliche Methode zur Trennung kann verwendet werden, um die Verbindung der Formel IA von der Verbindung der Formel IB zu isolieren. Unter den üblichen Methoden zur Trennung dieser zwei Verbindungen können Extraktion und Destillation genannt werden.

Die nach dem erfindungsgemässen Verfahren erhältlichen Verbindungen sind nützliche Zwischenprodukte zur Herstellung von Verbindungen zur Behandlung von Allergien und entzündlichen Erkrankungen, wie Kontaktdermatitis, Psoriasis und Entzündungen des Gastrointestinaltraktes. Solche anti-allergischen und anti-inflammatorischen Verbindungen sind auch als Leukotrien-Antagonisten, SRS-A (substance of anaphylaxis)-Antagonisten und Vermittler des 5-Lipoxygenase-Wegs bekannt. Verfahren zu deren Herstellung aus Verbindungen wie den erfindungsgemäss Herstellbaren sind in der Patentliteratur beschrieben, beispielsweise in der publizierten Europäischen Patentanmeldung Nr. 129906 vom 24. Juni 1983 [Chemical Abstracts, 103, 6223 S (1985)].

Die nachstehenden Beispiele illustrieren im weiteren die vorliegende Erfindung, ohne diese zu beschränken.

In diesen Beispielen wurde die enantiomere Reinheit (% e.R.) der R- und S-Ester mittels Hochdruck-Flüssigchromatographie (HPLC)-Analyse an einer 25 cm x 4,6 cm Kolonne mit kovalent gebundenem (R)-Phenylglycin (Regis Chromatography Co.) bestimmt. Diese Kolonne wurde mit 10% Aethanol/Heptan bei einer Durchflussgeschwindigkeit von 1 ml/Min. eluiert. Die eluierten Fraktionen wurden mit einem UV-Detektor bei 254 nm bestimmt. Für eine Beschreibung der Kolonne und der bekannten Auftrennungen vergleiche W.H. Pirkle et al., J. Org. Chem., 46, 1981, Seite 4988. Die Carbonsäuren wurden nach der Ueberführung in die entsprechenden Ester analysiert.

## Beispiel 1

### Enzymatisch kinetische Aufspaltung von racemischem 3,4-Dihydro-7-hydroxy-2H-1-benzopyran-2-carbonsäureäthylester

Ein 250 ml-Dreihalskolben, welcher mit einem mechanischen Rührwerk, einer mit einem pH-Kontrollgerät verbundenen pH-Elektrode und einer mit einer peristaltischen Pumpe verbundenen Zugabevorrichtung ausgerüstet ist, wird mit 60 ml deionisiertem Wasser, 15 ml 0,05M Phosphatpuffer (pH 7,0) und 2,2 g racemischem 3,4-Dihydro-7-hydroxy-2H-1-benzopyran-2-carbonsäureäthylester in 7,5 ml Tetrahydrofuran beladen. Man stellt den pH-Wert mit 0,1N wässriger Natriumhydroxidlösung auf 8,0 ein und gibt unter kräftigem Rühren 0,4 g Pseudomonas Lipase-Enzym (P-30, Amana International Enzyme Co., Inc., Troy, Virginia) zu. Um den Verlust des Co-Solvens durch Verdampfen zu vermeiden, verschliesst man das Reaktionsgefäss und rührt in gleicher Weise weiter. Durch Zugabe von 0,1N wässriger Natriumhydroxidlösung mit Hilfe der peristaltischen Pumpe wird der pH-Wert bei 8,0 gehalten. Man unterbricht die Reaktion, wenn 55 ml 0,1N wässrige Natriumhydroxidlösung verbraucht worden sind (nach ungefähr 10 Stunden), und

entfernt das Tetrahydrofuran durch Abdampfen bei 35°C und einem Druck von 1300 Pa. Das zurückbleibende Gemisch wird dreimal mit 50 ml (total 150 ml) Aethylacetat extrahiert. Die vereinigten organischen Extrakte werden mit 50 ml gesättigter wässriger Natriumbicarbonatlösung gewaschen und über wasserfreiem Natriumsulfat getrocknet. Die Lösung wird filtriert und bei 40°C und einem Druck von 1300 Pa eingedampft, wobei man 1,0 g (45%; 100% der Theorie) (R)-3,4-Dihydro-7-hydroxy-2H-1-benzopyran-2-carbonsäureäthylester als weisslichen Festkörper erhält, Schmelzpunkt 77-78°C, $[\alpha]_D^{25}$ = -20,2° (c 1,0, Chloroform), 99,6% e.R.

Die wässrige Phase wird mit konzentrierter Salzsäure auf pH 1,0 eingestellt und zweimal mit je 50 ml (total 100 ml) Aethylacetat extrahiert. Die vereinigten organischen Extrakte werden über wasserfreiem Natriumsulfat getrocknet, filtriert und bei einem Druck von 1300 Pa bei 40°C eingedampft, wobei man 0,96 g (50%; 90% der Theorie) (S)-3,4-Dihydro-7-hydroxy-2H-1-benzopyran-2-carbonsäure als weisslichen Festkörper erhält, Schmelzpunkt 156,5-158,5°C, $[\alpha]_D^{25}$ = -9,2° (c 1,0, Methanol), 75% e.R.

## Beispiel 2

Herstellung von (S)-3,4-Dihydro-7-hydroxy-2H-1-benzopyran-2-carbonsäureäthylester durch enzymatische Hydrolyse

Ein 5 l-Dreihalskolben, ausgerüstet mit einem mechanischen Rührwerk, einer mit einem pH-Kontrollgerät verbundenen pH-Elektrode und einer mit einer peristaltischen Pumpe verbundenen Zugabevorrichtung, wird mit 2,73 l deionisiertem Wasser, 682 ml 0,05M Phosphatpuffer (pH 7,0) und einer Lösung von 100,0 g (0,45 Mol) racemischem 3,4-Dihydro-7-hydroxy-2H-1-benzopyran-2-carbonsäureäthylester in 340 ml Tetrahydrofuran beladen. Der pH-Wert wird mit 1,0N wässriger Natriumhydroxidlösung auf 8 eingestellt, und 9,0 g Pseudomonas Lipase-Enzym (P-30, Amano International Enzyme Co., Inc., Troy, Virginia) wird zum Reaktionsgemisch gegeben. Die Hydrolyse erfolgt unter kräftigem Rühren und halten des pH-Werts bei 8 durch Zugabe von 1,0N wässriger Natriumhydroxidlösung mittels der Pumpe. Die Hydrolyse wird gestoppt, wenn 40% der Base aufgebraucht ist. Das Tetrahydrofuran im Reaktionsgemisch wird bei 40°C (1300 Pa) entfernt, und die wässrige Phase mit dreimal 1,0 l (3,0 l) Aethylacetat extrahiert. Die vereinigten organischen Extrakte werden nacheinander mit 500 ml gesättigter wässriger Natriumbicarbonatlösung und 500 ml gesättigter Kochsalzlösung gewaschen und dann über wasserfreiem Natriumsulfat getrocknet. Das Trocknungsmittel wird abfiltriert, und das Lösungsmittel bei 35°C (1300 Pa) entfernt, wobei man 35,1 g (35%, 86% der Theorie) (R)-3,4-Dihydro-7-hydroxy-2H-1-benzopyran -2-carbonsäureäthylester als beigefarbenen Festkörper erhält, Schmelzpunkt 77-78°C, $[\alpha]_D^{22}$ = -18,8° (c 1,0, Chloroform), 93,5% e.R.

Die vereinigten wässrigen Phasen werden mit 100 ml konzentrierter Salzsäure auf pH 1,0 eingestellt und mit dreimal 1,0 l (3,0 l) Aethylacetat extrahiert. Die vereinigten organischen Extrakte werden mit 500 ml gesättigter Kochsalzlösung gewaschen und über wasserfreiem Natriumsulfat getrocknet. Nach Abfiltrieren wird das Lösungsmittel bei 40°C (1300 Pa) eingedampft, wobei man 51,6 g rohe (S)-3,4-Dihydro-7-hydroxy-2H-1-benzopyran-2-carbonsäure in Form eines festen Gummis erhält. Eine kleine Probe dieses Materials (1,6 g) wird durch Anreiben mit Chloroform und Trocknen über Nacht bei 40-45°C (65 Pa) gereinigt, Schmelzpunkt 156,5-158,5°C, $[\alpha]_D^{22}$ = -12,1° (c 1,0, Methanol). Die verbleibende rohe Carbonsäure wird danach verestert, indem man diese in 1,0 l Aethanol, welches 1,0 ml konzentrierte Schwefelsäure enthält, unter Argon während 2 Stunden zum Rückfluss erhitzt. Der grösste Teil des Lösungsmittels wird bei Atmosphärendruck abdestilliert, und der Rückstand in 500 ml deionisiertes Wasser gegossen. Das Gemisch wird mit dreimal 100 ml (300 ml) Aethylacetat extrahiert. Die vereinigten organischen Extrakte werden nacheinander mit 200 ml gesättigter wässriger Natriumbicarbonatlösung und 200 ml deionisiertem Wasser gewaschen und über wasserfreiem Natriumsulfat getrocknet. Nach dem Abfiltrieren wird das Lösungsmittel bei 40°C (1300 Pa) entfernt, und der Festkörper über Nacht bei 40-45°C (65 Pa) weitergetrocknet. Es verbleiben 30,0 g (26%, 50% der Theorie) optisch aktiver (S)-3,4-Dihydro-7-hydroxy-2H-1-benzopyran -2-carbonsäureäthylester, Schmelzpunkt 77-79°C, $[\alpha]_D^{22}$ = +18,1° (c 1,0, Chloroform), 83% e.R.

Dieser Ester wird dann nochmals der enzymatischen Hydrolyse mit Pseudomonas Lipase (P-30, Amano International Enzyme Co., Inc., Troy, Virginia) unterworfen, um seine enantiomere Reinheit zu verbessern. Ein 3 l-Dreihalskolben, welcher wie oben beschrieben ausgerüstet ist, wird mit 775 ml deionisiertem Wasser, 194 ml 0,05M Phosphatpuffer (pH 7,0) und 28,4 g (0,128 Mol) des obigen Esters in 96 ml Tetrahydrofuran beladen. Der pH-Wert wird mit der entsprechenden Menge 1,0N wässriger Natriumhydroxidlösung auf 8 eingestellt, und 2,6 g Pseudomonas Lipase-Enzym werden zur Mischung gegeben. Man hydrolysiert unter kräftigem Rühren und Halten des pH-Werts bei 8 bis 70% im Zeitraum von 7 Stunden hydrolysiert sind. Das Tetrahydrofuran wird bei 40° C (1300 Pa) aus dem Reaktionsgemisch entfernt, und die wässrige Phase mit dreimal 250 ml (750 ml) Aethylacetat extrahiert. Die vereinigten organischen Extrakte werden nacheinander mit 250 ml gesättigter wässriger Natriumbicarbonatlösung und 250 ml deionisiertem Wasser gewaschen. Die organische Phase wird über wasserfreiem Natriumsulfat getrocknet, abfiltriert und bei 40° C (1300 Pa) eingedampft, wobei man 6,7 g von nahezu racemischem 3,4-Dihydro-7-hydroxy-2H-benzopyran-2-carbonsäureäthylester erhält. Die wässrigen Phasen werden mit 33 ml konzentrierter Salzsäure auf pH 1 gestellt und mit dreimal 250 ml (750 ml) Aethylacetat extrahiert. Die vereinigten organischen Extrakte werden mit 250 ml gesättigter Kochsalzlösung gewaschen und über wasserfreiem Natriumsulfat getrocknet. Nach Abfiltrieren wird das Lösungsmittel bei 40° C (1300 Pa) entfernt, wobei man 20,4 g (S)-3,4-Dihydro-7-hydroxy-2H-1-benzopyran-2-carbonsäure erhält, 95,5% e.R. Dieses Material wird wie oben beschrieben verestert, wobei man 21,6 g (76%) (S)-3,4-Dihydro-7-hydroxy-2H-1-benzopyran -2-carbonsäureäthylester als beigefarbenen Festkörper erhält, Schmelzpunkt 77-79° C, $[\alpha]_D^{22}$ = +19,1° (c 1,0, Chloroform), 95,5% e.R.

## Ansprüche

1. Verfahren zur Herstellung eines 2R-Esters der allgemeinen Formel

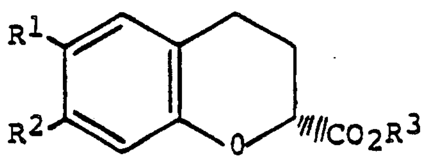

IA

und einer 2S-Carbonsäure der allgemeinen Formel

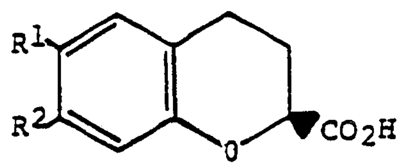

IB

worin einer von $R^1$ und $R^2$ Hydroxy und der andere Wasserstoff und $R^3$ Alkyl, Aryl oder Aralkyl bedeuten, dadurch gekennzeichnet, dass man einen racemischen 2RS-Ester der allgemeinen Formel

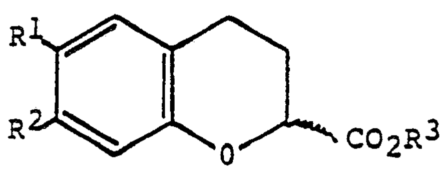

II

worin $R^1$, $R^2$ und $R^3$ die oben angegebene Bedeutung besitzen, mit einem bakteriellen Lipase-Enzym in einem wässrigen oder wässrig-organischen Reaktionsmedium zur selektiven Ueberführung des racemischen 2RS-Esters in den 2R-Ester und die 2S-Carbonsäure umsetzt, wobei die Umsetzung bei einem pH-Wert zwischen etwa 5 und etwa 10 durchgeführt wird, und danach den 2R-Ester und die 2S-Säure getrennt aus dem Reaktionsmedium isoliert.

2. Verfahren gemäss Anspruch 1, worin die Reaktion bei einem pH-Wert zwischen etwa 7 und etwa 9 durchgeführt wird.

3. Verfahren gemäss Anspruch 1 oder 2, worin das Reaktionsmedium aus Wasser und Tetrahydrofuran besteht.

4. Verfahren gemäss Anspruch 3, worin das Reaktionsmedium aus Wasser und Tetrahydrofuran im Verhältnis von etwa 3:1 bis 9:1 besteht.

5. Verfahren gemäss einem der Ansprüche 1-4, worin $R^3$ Niederalkyl bedeutet.

6. Verfahren gemäss Anspruch 5, worin $R^3$ Methyl oder Aethyl bedeutet.

7. Verfahren gemäss Anspruch 6, worin $R^1$ Wasserstoff, $R^2$ Hydroxy und $R^3$ Aethyl bedeuten.

8. Verfahren gemäss einem der Ansprüche 1-7, worin das bakterielle Lipase-Enzym aus einer Pseudomonas Spezies gewonnen wurde.